(19) Europäisches Patentamt
European Patent Office
Office européen des brevets

(11) Veröffentlichungsnummer: **0 152 612 B1**

(12) # EUROPÄISCHE PATENTSCHRIFT

(45) Veröffentlichungstag der Patentschrift: **21.02.90**

(51) Int. Cl.[5]: **G 01 N 33/531**, G 01 N 33/577, G 01 N 33/68 // C12P21/00, C12N15/00

(21) Anmeldenummer: **84116008.8**

(22) Anmeldetag: **20.12.84**

(54) **Bestimmung von Fibrin mit Fibrin-spezifischen Antikörpern.**

(30) Priorität: **09.01.84 DE 3400434**

(43) Veröffentlichungstag der Anmeldung: **28.08.85 Patentblatt 85/35**

(45) Bekanntmachung des Hinweises auf die Patenterteilung: **21.02.90 Patentblatt 90/8**

(84) Benannte Vertragsstaaten: **AT BE CH DE FR GB IT LI LU NL SE**

(56) Entgegenhaltungen:
**SCIENCE, Band 222, Nr. 4628, 9. Dezember 1983, Seiten 1129-1132, Washington, US; K.Y. HUI et al.: "Monoclonal antibodies to a synthetic fibrin-like peptide bind to human fibrin but not fibrinogen"**
**CHEMICAL ABSTRACTS, Band 100, Nr. 15, 9. April 1984, Zusammenfassung 119083c, Seite 427, Columbus, Ohio, US; B. KUDRYK et al.: "Specificity of a monoclonal antibody for the amino-terminal region of fibrin" & MOL. immunol. 1984, 21(1), 89-94**

(73) Patentinhaber: **Max-Planck-Gesellschaft zur Förderung der Wissenschaften e.V., Bunsenstrasse 10, D-3400 Göttingen (DE)**
Patentinhaber: **BEHRINGWERKE Aktiengesellschaft, Postfach 1140, D-3550 Marburg 1 (DE)**

(72) Erfinder: **Scheefers-Borchel, Ursula Dr., Ludwigstrasse 46, D-6301 Linden Grossen-Linden (DE)**
Erfinder: **Müller-Berghaus, Gert, Prof. Dr., Klein-Lindener-Strasse 31, D-6300 Giessen (DE)**
Erfinder: **Eberle, Reinhard. Dr., Stauffenbergstrasse 83, D-7400 Tübingen (DE)**
Erfinder: **Fuhge, Peter, Dr., Mühlenstrasse 8, D-3551 Lahntahl-Caldern (DE)**
Erfinder: **Heimburger, Norbert. Prof.Dr., Sonnenhang 10, D-3550 Marburg 1 (DE)**

(74) Vertreter: **Klein, Otto, Dr. et al, Hoechst AG Zentrale Patentabteilung Postfach 80 03 20, D-6230 Frankfurt am Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur selektiven Bestimmung von Fibrin im Plasma oder im Gewebe mit hochspezifischen Antikörpern, die hierfür benötigten Antikörper, ein Verfahren zu deren Herstellung und die hierfür eingesetzten Immunogene.

Es ist bekannt, dass bei Patienten mit disseminierter intravasaler Gerinnung (Verbrauchskoagulopathie) lösliches Fibrin im zirkulierenden Blut auftritt. Dieses Fibrin ist durch die Einwirkung proteolytischer Enzyme aus dem löslichen Plasmaprotein Fibrinogen entstanden. Lösliches Fibrin kann sich in Kapillaren und kleinen Blutgefässen zu Fibrinsträngen zusammenlagern, die licht- und elektronenmikroskopisch nachweisbar sind (Übersicht bei: Müller-Berghaus, G., Seminars in Thrombosis and Hemostasis 3: 209-246, 1977). Auch bei entzündlichen Erkrankungen kann im Gewebe intra- oder extravaskulär ein Material nachgewiesen werden, das sich von Fibrinogen ableitet. Es bestand deshalb die Aufgabe, ein Reagenz bzw. ein Verfahren zu entwickeln, um Fibrin von Fibrinogen unterscheiden zu können und Fibrin spezifisch und quantitativ zu bestimmen.

Es ist bekannt, dass proteolytische Enzyme, wie z.B. Thrombin, vom Fibrinogenmolekül sowohl intra- als auch extravasal Peptide abspalten, so dass Fibrinmonomer entsteht. Mehrere Fibrinmonomere können sich zu einem Fibrinpolymer, einem Fibringerinnsel, zusammenlagern, das dann für den Organismus, wenn dies sich intravasal ereignet, zu deletären Folgen führt. Es ist demnach erstrebenswert, sehr frühzeitig bei einem krankhaften Geschehen Fibrin nachzuweisen.

Bisher gibt es nur qualitative Methoden zum Nachweis von Fibrin, da sich Fibrin von Fibrinogen nur durch die Abspaltung der Fibrinopeptide unterscheidet. Die qualitativen bzw. semiquantitativen Methoden beruhen z.B. auf einer Fällung nach Zugabe von Ethanol (Godal, H.C., Abildgaard, U., Scand. J. Haematol. 3: 342-350, 1966), Protaminsulfat (Niewiarowski, S., Gurewich, V., J. Lab. Clin. Med. 77: 665-676, 1971) oder Agglutination von beschichteten Erythrozyten oder Latexpartikeln (Largo, R., Heller, V., Straub, P.W., Blood 47: 991-1002, 1976). Diese zitierten Methoden erlauben nicht zwischen Fibrin und Fibrinogen zu unterscheiden. Andere Methoden versuchen Fibrin vom Fibrinogen durch Chromatographie zu trennen (Alkjaersig, N., Fletscher, A., Burstein, R., Am. J. Obstet. Gynecol. 122: 199-209, 1975; Heene, D.L., Matthias, F.R., Thromb. Res. 2: 137-154, 1973). Auch diese Methoden sind nicht spezifisch, da Fibrin sich nicht völlig von Fibrinogen abtrennen lässt. Fibrinogen ist notwendig, um Fibrin in Lösung zu halten (Krell, W., Mahn, I., Müller-Berghaus, G., Thromb. Res. 14: 299-310, 1979).

Eine mögliche Methode, im gereinigten System Fibrin von Fibrinogen zu unterscheiden, ist die Bestimmung des N-terminalen Glycins (Kierulf, P., Godal, H.C., Scand. J. Haematol. 9: 370-372, 1972). Diese Methode ist jedoch kompliziert und benötigt grosse Mengen an Ausgangsmaterial, weswegen sie sich nicht als Test durchgesetzt hat. In einer kleinen Plasma- oder Gewebeprobe kann somit bisher Fibrin nicht von Fibrinogen unterschieden werden.

Erfindungsgemäss wird diese Aufgabe dadurch gelöst, dass Fibrin mit Hilfe hochspezifischer Antikörper bestimmt wird.

Aus Science, Band 222, Nr. 4628, Seiten 1129-1132 (1983) sind gegen Fibrin hochspezifische monoklonale Antikörper sowie ein Verfahren zur Bestimmung von Fibrin mit Hilfe dieser Antikörper bekannt. Diese Antikörper werden unter Verwendung eines Peptids aus der β-Kette von menschlichem Fibrin als Immunogen erzeugt.

Es wurde nun überraschenderweise gefunden, dass hochspezifische Antikörper gebildet werden, wenn man als Antigen ein Peptid einsetzt, das eine Aminosäuresequenz des Fibrins enthält, die durch Abspaltung von Fibrinopeptid A vom Fibrinmolekül freigelegt wird. Dies war um so überraschender, als frühere Immunisierungsversuche gegen das komplette Fibrinmolekül zu keinen Fibrin-spezifischen Antikörpern führten.

Die Bestimmung von Fibrin kann mit den erfindungsgemässen Antikörpern auch in Anwesenheit vergleichsweise hoher Konzentrationen von Fibrinogen nicht nur qualitativ, sondern sogar quantitativ erfolgen.

Antiseren, die die erfindungsgemässen Antikörper enthalten, werden erhalten, indem man in den Organismus von Versuchstieren als Antigen Peptide mit einer Teilaminosäuresequenz des Fibrins einbringt. Bevorzugt sind Peptide, die eine Aminosäuresequenz von 3 bis 12 Aminosäuren enthalten, die nach Abspaltung des Fibrinopeptids A vom N-terminalen Ende des Fibrinogenmoleküls freigelegt wird. Als vorteilhaft hat sich das Hexapeptid der Formel Gly-Pro-Arg-Val-Val-Glu erwiesen. Dieses wird in üblicher Weise, zweckmässig unter Einschaltung einer «spacer»-Gruppierung, an hierfür geeignete Trägerpeptide gebunden.

Die Oligopeptide, die die Aminosäuresequenz des Fibrins, insbesondere der terminalen Gruppen, die durch Abspaltung der Fibrinopeptide freigesetzt werden, nachahmen, werden in bekannter Weise synthetisiert. Geeignete Verfahren sind beispielsweise in E. Wünsch in Houben-Weyl, «Methoden der organischen Chemie», Band XV/1 und 2; The Peptides, Vol. 1, «Major Methods of Peptide Bond Formation», Academic Press (1979), oder «Perspectives in Peptide Chemistry», A. Eberle, R. Geiger and T. Wieland (Editors), S. Karger, Basel (1981), beschrieben.

Dabei werden Aminosäuren oder Peptidfragmente, deren funktionelle Gruppen, die nicht an der Reaktion teilnehmen sollen, in geeigneter Weise geschützt sind, zur Reaktion gebracht. Die freien Carboxygruppen von Aminosäurederivaten oder Peptidfragmenten werden in geeigneter Weise aktiviert und mit der Aminogruppe von Aminosäurederivaten oder teilgeschützten Peptidfragmenten umgesetzt.

Die Aktivierung kann mit DCCI, DCCI/HOBt, DCCI/Hydroxysuccinimid, $POCl_3$, Chlorameisensäureestern oder der Azid-Methode erfolgen. Bevorzugt verwendet man die DCCI/HOBt-Methode nach Chem. Ber. 103, 788 (1970).

Der Aufbau der teilgeschützten Oligopeptide Boc-Gly-Pro-Arg-Val-Val-Glu-ε-Capr-OH und Fmoc-Gly-Pro-Arg-Val-Val-Glu-HMDA-H kann stufenweise vom C-terminalen Ende her oder auch durch Fragmentkupplungen erfolgen. Ausserdem kommt stufenweiser Aufbau an einem löslichen oder unlöslichen polymeren Träger, wie beispielsweise Polyoxyethylen oder vernetztes Polystyrol, in Frage. Als Schutzgruppe für die alpha-Aminofunktion der Aminosäuren oder Peptidfragmente kommen Boc, Fmoc, Z, α,α-Dimethyl-3,5-dimethoxybenzyloxycarbonyl, 2-(Biphenylyl-(4))-propyl-(2)-oxycarbonyl und Trityl in Frage. Bevorzugt verwendet man Boc, Fmoc und Z.

Als Schutz für die alpha-Carboxygruppe von Aminosäuren und Peptidfragmenten verwendet man die Veresterung mit Alkanolen oder araliphatischen Alkoholen. Bevorzugt sind die Methyl-, Benzyl- und tert. Butylester.

Die Seitenkettenfunktionen der teilgeschützten Oligopeptide sind eine Carboxygruppe sowie eine Guanidinofunktion. Diese können entsprechend ihrer Reaktivität mit in der Peptidchemie üblichen Schutzgruppen substituiert sein. Vorzugsweise verwendet man zum Schutz der Carboxygruppe in Glu die Veresterung mit aliphatischen doer araliphatischen Alkoholen wie beispielsweise Methanol, tert. Butanol oder Benzylalkohol. Zum Schutz der Guanidinofunktion des Arginins kann sie protoniert sein oder durch $N^G$-Tosyl, $N^G$-Nitro, $N^G$-Adamantyloxycarbonyl und $N^G$-Benzyloxycarbonyl substituiert sein. Die Protonierung wird bevorzugt.

Der N-Terminus der verwendeten Oligopeptide muss zur eindeutigen Reaktionsführung bei der Kopplung an das Trägerprotein so geschützt sein, dass eine Abspaltung der Schutzgruppe am Peptid-Protein-Konjugat ohne Schädigung des Proteins erfolgen kann. Dazu eignen sich die Fmoc-Schutzgruppe, die unter alkalischen Bedingungen sowie die die 2-(Biphenylyl-(4))-propyl-(2)-oxycarbonyl-, α,α-Dimethyl-3,5-dimethoxybenzyloxycarbonyl-, Boc- und Tritylgruppe, die unter relativ milden sauren Bedingungen abgespalten werden können.

Als «spacer»-Gruppierung kommen die in der Peptidchemie üblicherweise verwendeten Brükkenglieder in Betracht, wie sie beispielsweise auch bei der Immobilisierung von Enzymen verwendet werden. Die entsprechenden Reagenzien enthalten zwei gleiche oder verschiedene reaktive Gruppen an den Enden einer Alkylengruppe mit beispielsweise 2 bis 8 Kohlenstoffatomen, wobei als reaktive Gruppen solche in Betracht kommen, die mit den funktionellen Gruppen der Aminosäuren eine kovalente Bindung eingehen können. Geeignet sind beispielsweise Dialdehyde wie Glutaraldehyd, Diisocyanate wie 1,6-Hexamethylendiisocyanat, Diamine wie 1,6-Hexamethylendiamin oder ω-Aminocarbonsäuren wie ε-Aminocapronsäure. Diese bifunktionellen Reagenzien werden – gegebenenfalls nach Aktivierung – in bekannter Weise einerseits mit dem Oligopeptid und andererseits mit dem Träger verknüpft.

Die Umsetzung der teilgeschützten Oligopeptide mit dem Trägerprotein erfolgt im Falle von Boc-Gly-Pro-Arg-Val-Val-Glu-ε-Capr-OH über eine Aktivierung der Carboxygruppe mit Hilfe eines wasserlöslichen Carbodiimids wie beispielsweise N-Ethyl-N′-(3-dimethylaminopropyl)-carbodiimid oder mit DCCI/Hydroxysuccinimid. Fmoc-Gly-Pro-Arg-Val-Val-Glu-HMDA-H wird mit Hilfe eines bifunktionellen Reagenzes, wie beispielsweise MBS, über die freie Aminogruppe gebunden. Die Abspaltung der Boc-Schutzgruppe am Peptid-Protein-Konjugat mit 1,2 N HCl/HAc ist nur bei RSA möglich. Die Abspaltung der Fmoc-Schutzgruppe mit 0,2 N NaOH gelingt bei vielen Trägerproteinen, wie beispielsweise RSA oder KLH.

Als Träger kommen grundsätzlich beliebige Proteine in Betracht. Zweckmässig werden solche Trägerproteine ausgewählt, die keine Kreuzreaktion verursachen. Geeignet sind beispielsweise Albumine wie Rinderserum-Albumin oder Haemocyanine wie Keyhole limpet hemocyanine.

Mit den so erhaltenen Antigenen werden Versuchstiere wie Mäuse, Ratten, Kaninchen oder Ziegen in bekannter Weise immunisiert und so Antiseren mit polyklonalen Antikörpern erhalten.

In einer bevorzugten Ausgestaltung werden in entsprechender Weise monoklonale Antikörper nach der Methode von G. Köhler und C. Milstein, Nature 256, 495–497 (1975) erzeugt. Hierzu können die bekannten Mausmyelomazellinien eingesetzt werden. Als besonders günstig hat es sich erwiesen, eine Zellinie zu benutzen, die selbst kein Immunglobulin produziert.

Die erfindungsgemässen polyklonalen und monoklonalen Antikörper reagieren nicht mit Fibrinogen, wohl aber mit des-A-Fibrin (Fibrin Typ I), des-AB-Fribrin (Fibrin Typ II), dem Peptidkonjugat und dem synthetischen Peptid. Diese Bindung erfolgt mit einer so grossen Empfindlichkeit und Spezifität, dass Nanogramm-Mengen an monoklonalen Antikörpern Nanogramm-Mengen an Antigenen (des-A-Fibrin und/oder des-AB-Fibrin) selbst in Gegenwart von Fibrinogen in physiologischer Konzentration (3 mg/ml) spezifisch erkennen können.

Vorstehend und in den folgenden Beispielen wurden die nachstehenden Abkürzungen verwendet:

| | |
|---|---|
| Arg | L-Arginin |
| Boc | t-Butyloxycarbonyl |
| ε-Capr | ε-Aminocapronsäure (bzw. davon abgeleiteter Rest) |
| DCCI | Dicyclohexylcarbodiimid |
| DCH | Dicyclohexylharnstoff |
| des-AA-Fibrin | Typ I |
| des-AABB-Fibrin | Typ II |

| | |
|---|---|
| DMF | Dimethylformamid |
| ELISA | Enzymimmunoassay (enzyme linked immunosorbent assay) |
| Fmoc | Fluorenylmethoxycarbonyl |
| Glu | L-Glutaminsäure |
| Gly | Glycin |
| HAc | Essigsäure |
| HMDA | 1,6-Hexamethylendiamin (bzw. davon abgeleiteter Rest) |
| HOBt | 1-Hydroxy-1H-benzotriazol |
| Ig | Immunglobulin |
| i.p. | intraperitoneal |
| i.v. | intravenös |
| KLH | Keyhole limpet hemocyanine (Haemocyanin einer marinen Napfschnecke) |
| MBS | m-Maleinimidobenzoyl-N-Hydroxysuccinimid-Ester |
| OBzl | Benzylester |
| OtBu | t-Butylester |
| PBS | Phosphatgepufferte Kochsalzlösung (phosphate buffered saline) |
| Pro | L-Prolin |
| RSA | Rinderserum-Albumin |
| s.c. | subcutan |
| TFA | Trifluoressigsäure |
| Val | L-Valin |
| Z | Benzyloxycarbonyl |

Herstellung der synthetischen Peptide Die Peptide der Beispiele 1 und 2 werden durch Elementaranalyse, Aminosäureanalyse und Dünnschichtchromatographie charakterisiert. Die chemische Reinheit wird durch Dünnschichtchromatographie in verschiedenen Lösungsmittelgemischen festgestellt. Die Racemisierung wird gaschromatographisch auf einer mit ®Chirasil-Val belegten Glaskapillarsäule nach J. Chromat. Sci. 15, 174 (1977) überprüft und liegt für jede Aminosäure unter 2 Prozent.

Beispiel 1

Unter Einbeziehung von ε-Aminocapronsäure als Spacer wird das teilgeschützte Peptid Boc-Gly-Pro-Arg-Val-Val-Glu-ε-Capr-OH nach dem Syntheseschema I synthetisiert.

Beispiel 2

Unter Einbeziehung von Hexamethylendiamin als Spacer wird das teilgeschützte Peptid Fmoc-Gly-Pro-Arg-Val-Val-Glu-HMDA-H nach dem Syntheseschema II synthetisiert.

Beispiel 3

20 mg Boc-Gly-Pro-Arg-Val-Val-Glu-ε-Capr-OH (Beispiel 1) werden in 0,4 ml Ethanol gelöst und 4 mg DCCI sowie 2,5 mg Hydroxysuccinimid zugegeben. Nach 2 Stunden Rühren bei Raumtemperatur wird der Peptid-Hydroxysuccinimidester langsam zu einer Lösung von 760 mg RSA (reinst) in 7 ml Puffer (0,25 m HEPES, 0,2 m $CaCl_2$, pH 7,5) zugetropft. Nach 20 Stunden bei Raumtemperatur wird vom DCH abfiltriert, gegen destilliertes Wasser dialysiert und lyophilisiert.

300 mg des so erhaltenen Peptid-RSA-Konjugats werden in 1 ml 1,2 N HCl/HAc gelöst und 20 Minuten bei Raumtemperatur gerührt. Das Lösemittel wird im Vakuum abdestilliert, der Rückstand in Wasser gelöst und lyophilisiert.

Beispiel 4

20 mg KLH werden in 1 ml 0,05 m Natriumphosphatpuffer (pH 6) gelöst. Dann werden 3 mg MBS, gelöst in 0,2 ml DMF, zugegeben und 30 Minuten bei Raumtemperatur gerührt. Niedermolekulare Bestandteile werden durch Gelfiltration auf Sephadex® G 25 entfernt. Die Proteinfraktionen werden vereinigt und durch Ultrafiltration auf 4 ml konzentriert. Nach Zugabe von 10 ml Natriumboratpuffer (pH 9) werden 5 mg Fmoc-Gly-Pro-Arg-Val-Val-Glu-HMDA-H (Beispiel 2), gelöst in 0,5 ml DMF, zugetropft und die Lösung 3 Stunden bei Raumtemperatur gerührt. Zur Abspaltung der Schutzgruppe werden 15 ml 0,2 N NaOH zugegeben und 30 Minuten bei Raumtemperatur gerührt. Nach Neutralisation wird gegen destilliertes Wasser dialysiert und lyophilisiert.

Herstellung heterologer Antikörper

Beispiel 5

Ein an ein Trägerprotein (RSA, KLH) uni-direktional gekoppeltes Peptid wird in PBS gelöst und zu gleichen Teilen mit Freund-Adjuvans gemischt. Jeweils 200 µg dieses Gemisches werden in 6–8 Wochen alte Balb/c Mäuse i.p. und s.c. injiziert. Diese Injektionen werden zweimal im Abstand von 3–4 Wochen wiederholt. Antiseren der Mehrzahl so behandelter Tiere zeigen im ELISA-Test eine positive Reaktion gegen Fibrinmonomere und das unkonjugierte Peptid, aber keine Immunantwort gegen Fibrinogen.

Des-AA- und des-AABB-Fibrinmonomere werden durch Behandlung einer Fibrinogenlösung mit Batroxobin (2,5 U/mg Fibrinogen) bzw. Thrombin (5 U/mg Fibrinogen) hergestellt. Die entstandenen Gerinnsel werden isoliert und in 0,05 M Tris-Puffer (pH 7,4) mit 3-molarem Harnstoffzusatz gelöst.

Eine Repolymerisation erfolgt dadurch, dass der Harnstoff durch Dialyse entfernt wird. Das Gerinnsel wird wieder in dem zuvor beschriebenen Tris-Harnstoff-Puffer aufgelöst.

Die Gegenwart von Antikörpern im Mausserum gegen bestimmte Antigene (Fibrinogen, des-A-Fibrin (Fibrin Typ I), des-AB-Fibrin (Fibrin Typ II), unkonjugiertes Peptid) wird mittels ELISA bestimmt. Das Prinzip beruht darauf, dass eine Serumverdünnung mit den an einen Träger immobilisierten Antigenen inkubiert wird. Nach einem Waschschritt und anschliessendem Zusatz einer mit einem Enzym wie Phosphatase oder Peroxydase markierten anti-Maus-Ig-Lösung wird durch Zugabe eines Substrates eine Änderung der optischen Dichte in nur den Reaktionsgefässen gemessen, in denen die Serumantikörper mit den entsprechenden Antigenen reagiert haben.

Unter Anwendung eines ELISA konnten folgende optische Dichten mit Antiseren von Mäusen, die gegen ein synthetisches Hexapeptid immunisiert worden waren, gemessen werden:

Verwendete Antigene

| Maus | Fibrinogen | des-AA-Fibrin | des-AABB-Fibrin | unkonjugiertes Hexapeptid |
|---|---|---|---|---|
| 1 | 0 | 1,45 | 1,45 | 0,691 |
| 2 | 0,046 | 1,35 | 1,08 | 0,51 |
| 3 | 0,017 | 1,64 | 1,25 | 0,49 |
| 4 | 0,03 | 2,0 | 1,55 | 0,51 |

Dieses Ergebnis zeigt, dass heterologe Antikörper in Seren von Mäusen, die gegen konjugiertes Peptid immunisiert worden waren, eindeutig Fibrinogen und Fibrin unterscheiden können.

Herstellung monoklonaler Antikörper

Beispiel 6

Nach oben genanntem Schema mit konjugiertem Peptid (RSA, KLH) immunisierte Mäuse erhalten 3 Tage vor Entnahme der Milz eine i.v. Injektion von 100 µg konjugiertem Peptid, welches in PBS gelöst wird.

Etwa $10^8$ Zellen von der Milz einer immunisierten Maus werden mit $5 \cdot 10^7$ Myelomazellen (x63-Sp8-653, eine Linie, die kein Immunglobulin synthetisiert, zu beziehen bei The Salk Institute, Cell Distribution Center, San Diego CA 92112, USA) in Gegenwart von Polyethylenglykol (MG 3400) fusioniert. Fusionierte Zellen werden auf 4 Platten, die jeweils 24 Vertiefungen enthalten, ausgesät. Jede dieser Vertiefungen enthält $5 \cdot 10^7$ Milzzellen unimmunisierter syngener Mäuse in Nährmedium, welches Hypoxanthin, Aminopterin und Thymidin enthält.

Die Antikörper enthaltenden Überstände dieser fusionierten Zellen (Hybridoma) werden 10–14 Tage später mittels des oben beschriebenen ELISA-Tests bezüglich ihrer Spezifität gegen folgende Antigene getestet: Fibrinogen, des-AA-Fibrin, des-AABB-Fibrin und unkonjugiertes Peptid.

Um monoklonale Antikörper zu erhalten, die nur gegen Fibrinomonomer und gegen das synthetische Peptid gerichtet sind, werden Hybridomazellen, deren Überstände keine gegen Fibrinogen gerichteten Antikörper enthalten, zweimal kloniert.

Syntheseschema I

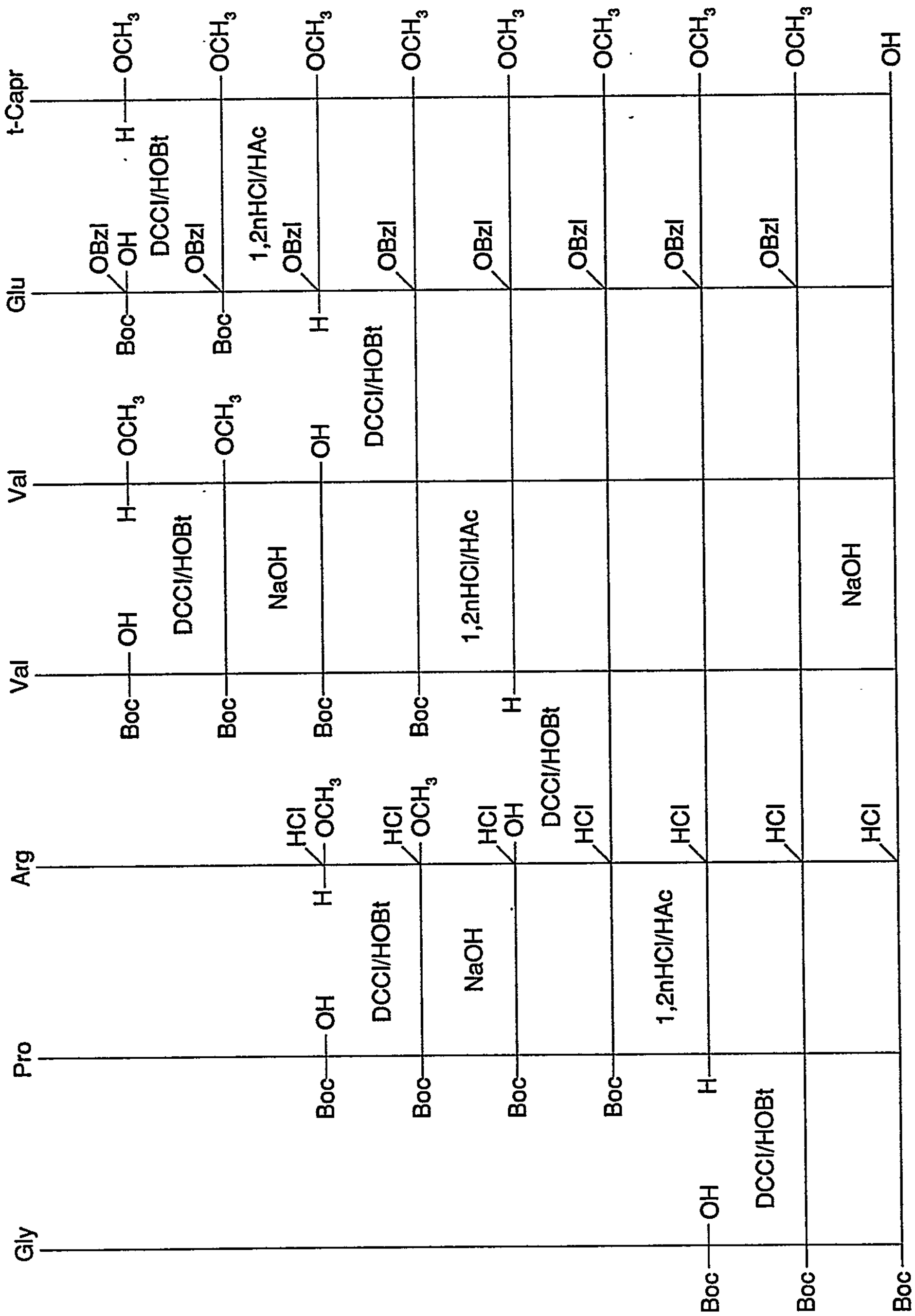

Syntheseschema II

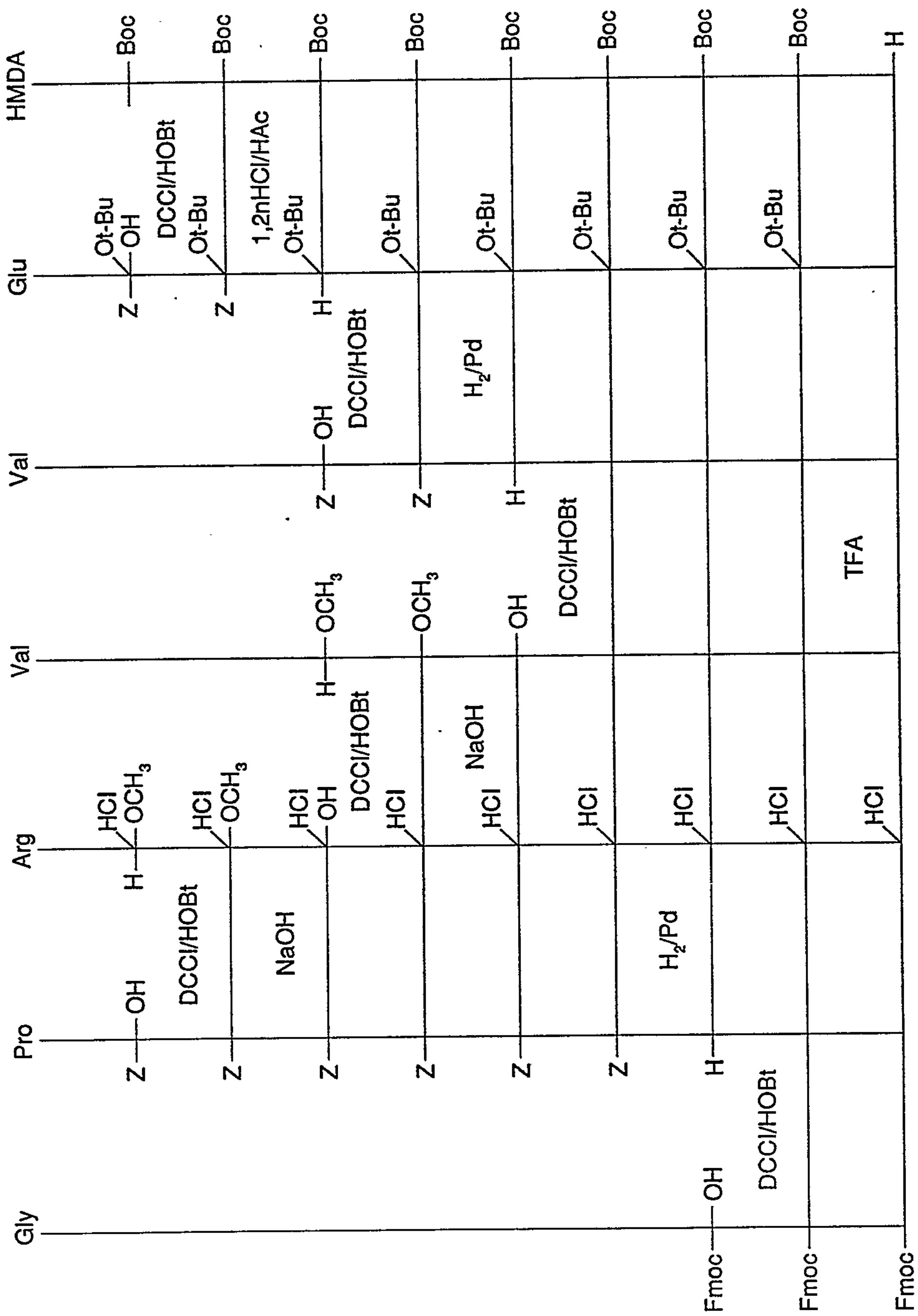

**Patentansprüche**

1. Gegen Fibrin hochspezifische Antikörper, erhältlich durch Immunisieren mit Hilfe eines Peptids, das eine Aminosäuresequenz des Fibrins enthält, die durch Abspaltung von Fibrinopeptid A vom Fibrinmolekül freigelegt wird.

2. Antikörper nach Anspruch 1, dadurch gekennzeichnet, dass die Aminosäuresequenz 3 bis 12 Aminosäuren enthält.

3. Antikörper nach mindestens einem der Ansprüche 1 und 2, dadurch gekennzheichnet, dass das Peptid die Sequenz

Gly-Pro-Arg-Val-Val-Glu

enthält.

4. Antikörper nach mindestens einem der Ansprüche 1 bis 3, dadurch gekennzeichnet, dass das Peptid die Aminosäuresequenz über eine Spacer-Gruppierung an ein Trägerpeptid gebunden enthält.

5. Antikörper nach mindestens einem der Ansprüche 1 bis 4, dadurch gekennzeichnet, dass sie monoklonal sind.

6. Verwendung eines Antikörpers nach mindestens einem der Ansprüche 1 bis 5 zur Bestimmung von Fibrin.

**Claims**

1. A highly specific antibody against fibrin, obtainable by immunization using a peptide which contains an amino acid sequence of fibrin which is exposed by splitting off fibrinopeptide A from the fibrin molecule.

2. An antibody as claimed in claim 1, wherein the amino acid sequence contains 3 to 12 amino acids.

3. An antibody as claimed in at least one of claims 1 and 2, wherein the peptide contains the sequence Gly-Pro-Arg-Val-Val-Glu.

4. An antibody as claimed in at least one of claims 1 to 3, wherein the peptide contains the amino acid sequence bound to a carrier peptide via a spacer group.

5. An antibody as claimed in at least one of claims 1 to 4, which is monoclonal.

6. The use of an antibody as claimed in at least one of claims 1 to 5 for determining fibrin.

**Revendications**

1. Anticorps hautement spécifiques contre la fibrine, pouvant être obtenus par immunisation à l'aide d'un peptide contenant une séquence d'aminoacides de la fibrine, qui est libérée par séparation du fibrinopeptide A d'avec la molécule de fibrine.

2. Anticorps selon la revendication 1, caractérisés en ce que la séquence d'aminoacides contient de 3 à 12 aminoacides.

3. Anticorps selon au moins l'une des revendications 1 et 2, caractérisés en ce que le peptide contient la séquence

Gly-Pro-Arg-Val-Val-Glu.

4. Anticorps selon au moins l'une des revendications 1 à 3, caractérisés en ce que le peptide contient la séquence d'aminoacides liée à un peptide de support par l'intermédiaire d'un groupement espaceur.

5. Anticorps selon au moins l'une des revendications 1 à 4, caractérisés en ce qu'ils sont monoclonaux.

6. Utilisation d'un anticorps selon au moins l'une des revendications 1 à 5, pour la détermination de la fibrine.